# EUROPEAN PATENT APPLICATION

(11) **EP 1 252 904 A2**
(43) Date of publication of application: **30.10.2002**
(21) Application number: 02009166.6
(22) Date of filing: 24.04.2002
(51) Int. Cl.: A61L 27/32, A61L 27/30

(54) **Precursors for active materials, active materials using such precursors, and method for producing said active materials**

(30) Priority: 25.04.2001 JP 2001128093
(71) Applicant: NAGOYA UNIVERSITY, Chikusa-ku Nagoya City Aichi Prefecture (JP)
(72) Inventor: Sugimura, Hiroyuki, Nagoya City, Aichi Pref. (JP); Takai, Osamu, Nagoya City, Aichi Pref. (JP); Gomez-Vega, Jose Manual, Nagoya City, Aichi Pref. (JP)
(74) Representative: Henkel, Feiler, Hänzel

(57) **Abstract**

A precursor for an active material, said precursor comprising an inorganic oxide and having hydroxide groups on a surface thereof, said hydroxide group being in such an amount as to precipitate an inorganic component in a reactive solution containing said inorganic component when the precursor is immersed in the solution.

## Description

### Background of the Invention

### Field of the Invention

The present invention relates to precursors for active materials, active materials using said precursors, and a method for producing said active materials. In particular, the invention relates to precursors of active materials in which an inorganic material is formed on a surface of an inorganic oxide, active materials using said precursors and a method for producing such active materials.

### Related Art Statement

Inorganic oxide porous bodies have been expected to be applied to various fields for absorption, separation, etc. Ordinarily, such porous bodies are classified into micro-porous solids having pore diameters of not more than 2 nm, mesoporous solids having pore diameters of 2 to 50 nm and macroporous solids having pore diameters of not more than 50 nm. If pore diameters of such porous bodies can be arbitrarily and finely controlled, they are expected to be applied to selective absorbents, catalyst materials, etc.

Since the ceramic materials are chemically stable among inorganic materials, they are utilized in various active materials including alumina, apatite hydroxide, tribasic calcium phosphate, bioactive glass, etc. Such active materials can be used as materials replacing living hard tissues such as dental materials or artificial bone materials. The active materials are sometimes used in the state that they are coated on substrates such as titanium, niobium or tantalum to enhance their strength.

In general, in order to produce inorganic oxide porous bodies as active materials, a method is known, which comprises the steps of forming an inorganic-organic structure and then removing the organic material in the structural body by extracting it with a solvent, thermally treating the structural body in air or oxygen or treating it with an acid. Precursors obtained by this method for active materials are thus known.

Among the ceramic materials, apatite hydroxide is an extremely promising material as an artificial bone material because of its high affinity to living bodies. On the other hand, apatite hydroxide is an extremely brittle material. Therefore, in order to produce an active material such as apatite hydroxide, a method is known, which produces an active material by coating apatite hydroxide on the surface of a structural material such as a titanium alloy.

In order to improve adhesion between the apatite including apatite hydroxide and a substrate, a method is also known, which once coats an inorganic material such as silica on the surface of the substrate, and precipitates apatite hydroxide on the surface of the silica from a simulated body fluid.

However, in the method for producing the porous body by removing the organic material through the heating treatment or the acid treatment as mentioned above, it is feared that cracks or peeling are likely to occur, because stress is formed inside the material. Further, since the heating treatment or the acid treatment is carried out, it is very difficult to coat the inorganic material on a substrate material having inferior heat resistance, for example, plastics.

Further, since apatite exhibits poor adhesion when coated at a low temperature, the coating is formed by a high temperature process such as flame spray coating or sintering. Consequently, there is a fear that the affinity to living bodies of the coated apatite hydroxide is modified to deteriorate the living affinity. In addition, since the high temperature process is used, this method is unfavorably used only for substrates having high heat resistance, for example, titanium alloys or the like.

Furthermore, the method in which the inorganic material is once coated on the surface of the substrate and then apatite hydroxide is precipitated has problems in that it takes a time to perform the precipitation due to poor activity of silica and the coated density is insufficient.

Besides, the activity of precursors for the above-mentioned active materials is very insufficient, so that it is difficult to precipitate the inorganic material merely by immersing the precursor in a reactive solution. For these reasons, neither active materials themselves using the above precursors nor active materials having desired characteristics such as sufficient activity have not been obtained.

Therefore, development of a method for producing new active materials, which method can be applied to not only substrates having high heat resistance but also those having low heat resistance as well as precursors, for the active materials, upon which inorganic materials can be easily precipitated have been desired.

### Summary of the Invention

It is an object of the present invention to provide precursors, for active materials, upon which an inorganic material can be easily precipitated, active materials using such precursors, and a method for enabling the production of the active materials at low temperatures irrespective of kinds of the substrates.

In order to accomplish the above objects, the present inventors made strenuous studies on the removal of organic materials in various inorganic-organic structural bodies and the activity of inorganic oxide bodies obtained. As a result, the inventors discovered the invention.

The precursor for the active material according to the present invention is characterized in that the precursor is a precursor, for the active material, which precursor comprises an inorganic oxide and has hydroxide groups on a surface thereof, said hydroxides group being in such an amount as to precipitate an inorganic component in a reactive solution containing said inorganic component when the precursor is immersed in the solution.

The reactive solution contains phosphate ions and calcium ions, for example. The mechanism of the precipitation reaction when the precursor is immersed in the solution is unknown. It may be considered, however, in the case of the phosphate ions and the calcium ions that the calcium ions are first absorbed on the surface of the precursor and the reaction continues between the absorbed calcium ions and the phosphate ions. Alternatively, it may be considered that clusters and fine crystals are formed between the phosphate ions and the calcium ions in the reaction solution, and they precipitate on the surface of the precursor. In order to cause the precipitate to cover the entire surface of the precursor, it is considered that the hydroxide groups are present on the surface of the precursor at a rate of at least one hydroxide per 1 nm².

The precursor according to the present invention is preferably a porous body. Such a porous body may be a micro-porous solid having pore diameters of not more than 2 nm, a mesoporous solid having pore diameters of 2 to 50 nm or a macroporous solid having pore diameters of not more than 50 nm. The micro-porous solid, the mesoporous solid or the macroporous solid may be selectively produced by appropriately adjusting the mixing ratio of the starting materials, producing conditions, etc. Such adjustment will be easily made by the skilled person in the art to which the invention pertains.

In a preferred embodiment of the precursor for the active material according to the present invention, the inorganic oxide is a ceramic material or a precursor of such a ceramic material.

In a further preferred embodiment of the precursor for the active material according to the present invention, the ceramic material or the ceramic material precursor is at least one selected from the group consisting of a silica-based material, a titania-based material and an alumina-based material.

In a further preferred embodiment of the precursor for the active material according to the present invention, the precursor is formed in a filmy shape.

In another preferred embodiment of the precursor for the active material according to the present invention, the inorganic oxide is obtained by removing the organic material from an inorganic oxide-organic molecular structure through optical oxidation. The inorganic oxide-organic molecular structure will be explained later in detail.

In a further preferred embodiment of the precursor for the active material according to the present invention, the reactive solution is a aqueous mixed solution of NaCl, KCl, CaCl₂, MgCl₂, NaH₃PO₄, and (CH₂OH)₃CNH₂.

For example, the aqueous solution may contain 142 mM of NaCl, 5 mM of KCl, 2.5 mM of CaCl₂, 1.5 mM of MgCl₂, 5mM of NaH₃PO₄ and 50 mM CH₂OH)₃CNH₂. There are many phosphate and carbonate species that can be formed. SBF (stimulated body fluid) is supersaturated in HPO₄², but at pH between 9.5 and 10.5 precipitation of apatite would take place spontaneously. At SBF (pH=7.25), it is necessary to cause a nucleation catalyzed by an appropriate surface to induce the formation of apatite. It was reported by de Aza et al. that the solubility diagrams and the ranges of the solubility of the different species depended upon pH and concentrations (See P.N. de Aza, F. Guitian, A. Merios,E. Lora-Tamayo, S. de Aza, "Bioceramics-simulated body fluid interfaces : pH and its influence of hydroxylapatite formation", J. Mater. Sci. Mater. Med. 7 (1996) pp 399-402.

In a still further preferred embodiment of the precursor for the active material according to the present invention, the inorganic component is apatite.

The present invention also relates to an active material comprising the above precursor and an inorganic material formed on a surface of the precursor by immersing the precursor into the reactive solution.

The present invention also relates to a method for producing an active material, comprising the steps of preparing an inorganic oxide-organic molecular structure by mixing an inorganic oxide with organic material selectively removing the organic material of said structure by optical oxidation, immersing a precursor for said active material comprising the inorganic oxide obtained thereby into a reactive solution, and thereby forming an inorganic material on a surface of precursor.

The inorganic material covers the surface of the precursors. When the precursor is a porous body, the inorganic material enters pores and cover inner surfaces of the pores. Assuming that the volume of the pores amounts to 50 vol. % of the precursor, for example and that no great difference in density exists between apatite and silica, for example, 1 g of the apatite may precipitate inside the pores of 1 g the porous body, provided that no apatite precipiates on the surface of the porous body.

In a preferred embodiment of the active material-producing method according to the present invention, the optical oxidation is effected by irradiation with at least one of ultraviolet rays under vacuum, ozone and plasma under vacuum.

In another preferred embodiment of the active material-producing method according to the present invention, the optical oxidation is effected by irradiating the inorganic oxide-organic molecular structure with said ultraviolet rays under vacuum.

In a further preferred embodiment of the active material-producing method according to the present invention, the reactive solution is a aqueous mixed solution containing NaCl, KCl, CaCl₂, MgCl₂, NaH₃PO₄, and (CH₂OH)₃CNH₂.

In a still further preferred embodiment of the active material-producing method according to the present invention, the inorganic material is apatite hydroxide.

In a still further preferred embodiment of the present invention, the inorganic material is a ceramic material or a precursor of the ceramic material.

In a still further preferred embodiment of the present invention, the ceramic material or the ceramic material precursor is at least one selected from the group consisting of a silica-based material, a titania-based material and an alumina-based material.

In a still further preferred embodiment of the present invention, the precursor is formed in a filmy shape.

In a still further preferred embodiment of the present invention, the filmy precursor is formed by at least one method selected from the group consisting of a sol-gel method, a CVD method and a chemical deposition method.

These and other objects, features and advantages of the invention will be appreciated upon reading the following description of the invention when taken in conjunction with the attached drawings, with the understanding that some modifications, variations and changes could be easily made by the skilled person in the art to which the invention pertains.

### Brief Description of the Drawings

For a better understanding of the invention, reference is made to the attached drawings, wherein:
Fig. 1 (a) gives an EDX spectrum of an active material obtained by the present invention, Fig. 1(b) a FTIR spectrum of an active material obtained by the present invention, and Fig. 1(c) and 1(d) are microscope photographs of the active materials obtained in the present invention.
Fig. 2 gives an IR spectrum of the precursor for the active material according to the present invention. Fig. 3 gives an IR spectrum of a precursor of an active material in Comparative Example.

### Detailed Description of the Invention

The precursor for the active material according to the present invention is an active material-precursor which comprises an inorganic oxide and has hydroxide groups on a surface thereof, the hydroxide groups being in such an amount as to precipitate an inorganic component in a reactive solution containing said inorganic component when the precursor is immersed in the solution.

The inorganic oxide to constitute the precursor is not limited to any particular material so long as it can form an inorganic skeleton for the active material. As such an inorganic oxide, mention may be made of ceramic materials and precursors for the ceramic materials, for example. As the ceramic materials and the precursors thereof, mention may be made of silica-based materials, titania-based materials and alumina-based materials may be recited, for example.

The precursor for the active material according to the present invention has a large amount of hydroxide. This amount of the hydroxide groups is such an amount as to precipitate an inorganic component in a reactive solution containing said inorganic component onto the precursor when the precursor is immersed in the solution. As mentioned above, a high active material having a highly covering density of the inorganic material having sufficient activity can be obtained owing to the hydroxide groups.

The inorganic oxide to form the precursor is preferably obtained by removing the organic material from the inorganic oxide-organic molecular structure through the optical oxidation. The method for removing the organic material will be explained in detail in connection with the method for producing the active material as mentioned later.

According to the active material of the present invention, the inorganic material is formed on the surface of the inorganic oxide by immersing the precursor therefor into the reactive solution. Therefore, the active material can be easily obtained by using and merely immersing the precursor in the solution. For example, when a mixed aqueous solution of NaCl, KCl, CaCl₂, MgCl₂, NaH₃PO₄ and (CH₂OH)₃CNH₂ is used as the reactive solution, apatite is precipitated as the inorganic component, so that the active material having apatite hydroxide can be obtained.

In the following, the precursor for the active material as well as the active material according to the present invention will be explained in detail with reference to the production method thereof.

In the active material-producing method according to the present invention, the inorganic oxide-organic molecular structure is formed by mixing the organic oxide with the organic material, and selectively removing the organic material from the structural body by optical oxidation.

First, the method for producing the inorganic oxide-organic molecular structure will be explained. The structural body is obtained by mixing the inorganic oxide with the organic material. The inorganic oxide is used to form the inorganic skeleton of the active material. As such an inorganic oxide, ceramic materials and precursors therefor may be recited, for example, judging from the fact that materials having mechanical strength are preferred. As the ceramic materials and the precursors therefor, silica-based materials, titania-based materials and alumina-based materials may be recited.

In order to form a silica skeleton with use of the silica-based material, a silicon-containing organic reagent including tetramethoxy silane, tetraethoxy silane, tetrabutoxy silane, methyltrimethoxy silane, methyltriethoxy silane, methyl tributoxy silane, tetrachloro silane, methyl trichloro silane, etc. may be arbitrarily used.

When a titania skeleton is formed with use of the titania-based material, titanium-containing organic reagents including tetraisopropoxide, titanium-n-butoxide, titanium chloride triisopropoxide, titanium dichloride diethoxide, titanium ethoxide and titanium isobutoxide may be arbitrarily used.

When an alumina skeleton is formed with use of an aluminum-based material, aluminum-containing organic reagents such as aluminum (II) n-butoxide, aluminum (III) s-butoxide, aluminum (II) t-butoxide, aluminum (III) ethoxide, aluminum (III) isoproxide may be arbitrarily used.

Next, the organic material will be explained. The organic molecules of the organic material have a role to impart porosity upon the active material. The organic material is finally scattered off and disappears from the inorganic oxide-organic molecular structure through optical oxidation. Thereby, a number of pores can be formed at locations of the inorganic oxide-organic molecular structure where the organic material is removed. Therefore, the organic material is not particularly limited, so long as it can be selectively removed by the optical oxidation.

As such an organic material, mention may be made of silane coupling agents having alkyl groups and perfluoroallkyl group, block copolymers, and surface active agents such as cetyl trimethyl ammonium salt and dodecyl sulfonic acid may be recited. The block copolymer is preferred as the organic material in that its organic molecules can form micells in the reactive solution. More preferably, a block copolymer composed of three blocks : a polyethylene oxide-polypropylene-polyethylene oxide. Further, a cationic surface active agent such as cetyl trimethyl ammonium salt can be also used.

The inorganic oxide-organic molecular structure may be produced in the form of a film or fine particles, for example. First, the production of the inorganic oxide-organic molecular structural body in a film form will be explained. A liquid raw material mixture is obtained by mixing an organic material, an inorganic raw material, hydrochloric acid and water. In this case, hydrochloric acid is used to gel Si alkoxide, for example. The mixing molar ratio of the organic material : the inorganic raw material : hydrochloric acid : water may be 0.01 ∼ 0.1 : 0.1 ∼ 5 : 1 ∼ 10 : 50 ∼ 500, for example. Preferably, it may be 0.01 ∼ 0.03 : 0.5 ∼ 1.5 : 4 ∼ 8 : 150 ∼ 200. Porous bodies having desired pores, that is, microporous solids; mesoporous solids, macroporous solids, etc. may be produced by changing the molar ratio of the organic material hydrochloric acid and water.

The inorganic oxide-organic molecular structural body can be formed on a substrate from the liquid raw material mixture by using at least one method selected from the sol-gel method, the CVD method and the chemical precipitation method. As the method for depositing the structural body on the substrate, the sol-gel method is preferable from the standpoint that the film can be formed near room temperature.

No particular limitation is posed upon the substrate to be used in the present invention. For, since the method for producing the active material according to the present invention can be carried out in such a low temperature range over the entire steps up to the final production as to afford no adverse effect upon the substrate, excluding the CVD, the substrate needs not be limited to a heat-resistant material, etc. Although not limited, light-weight structural materials including heat-resistant materials such as titanium, silicon, oxides such as alumina and mica, graphite, carbon fibers, plastics, etc. may be recited as the substrate. In general, single crystals of such as silicon, alumina and titania, mica, graphite, etc. are preferably used from the standpoint that their cleaved faces are smooth.

The inorganic oxide-organic molecular structural body in the form of fine particles can be produced as follows. As in the production of a thin film of the inorganic oxide-organic molecular structure, a liquid raw material mixture is obtained similarly by mixing an organic material, an inorganic raw material, hydrochloric acid and water at a given ratio, and fine powder is obtained as the inorganic oxide-organic molecular structural body by evaporating the liquid raw material mixture to dryness.

The organic material is removed from the inorganic oxide-organic molecular structural body formed above. The organic material may be removed by optical oxidation. The meaning of the optical oxidation is not particularly limited. Further, a light source for the optical oxidation is not particularly limited, either. As the light source for the optical oxidation, ultraviolet light source, zone light source, plasma light source, etc. may be recited. The structural body can be optically oxidized by applying the light beam upon it from the above light source. Preferably, vacuum ultraviolet rays having short wavelengths may be used. As the ultraviolet rays having such short wavelengths, an eximer lamps may be recited. The reason why the eximer lamp is recited is that vacuum ultraviolet rays of 172 nm have high photon energy, and can easily cleave the bonds of the organic material.

Although the light-irradiating time period is not particularly limited, it is ordinarily 1 ∼ 10 hours, preferably 2 ∼ 4 hours, when the eximer lamp is used. The reason why the light-irradiating time period is limited to this range is that the organic material can almost completely be removed. The light-irradiating time period may be varied depending upon the activity of the desired active material. In order to obtain the active material having higher activity, the light-irradiating time period may be prolonged.

Although the temperature is not particularly limited, a low temperature of not more than 200°C is preferred from the standpoint of increasing the density of the hydroxide groups. When the plastic substrate is used, the irradiation may be effected at a temperature of not more than 100°C.

The above light irradiation cleaves C-C bonds and C-H bonds in the organic material as the template, so that the organic material is scattered and disappears in the form of carbon dioxide and/or water through chemical reaction with activated oxygen. Thereby, the inorganic porous body having a porous structure composed of an inorganic skeleton is formed.

The inorganic oxide product obtained as the precursor by removing the organic material from the structural body is immersed into the reactive solution, thereby obtaining the active material as mentioned below. The reaction proceeds as the temperature becomes higher. However, the reaction is effected in the aqueous reactive solution, for example, the reaction temperature is in a range of 0 to 100°C. In the following Examples, the reaction was carried out at 37°C.

A large amount of hydroxide groups are formed on the inorganic oxide body obtained by removing the organic material from the above inorganic oxide-organic molecular structural body. The active material can be obtained owing to such a large amount of the hydroxide groups. When the inorganic oxide body is immersed into the reactive solution, the inorganic component in the reactive solution can be precipitated on the surface of the inorganic oxide due to the presence of a number of the hydroxide groups.

The hydroxide groups are considered to be formed as follows. That is, the outermost surface of the oxide is hydrolyzed to form hydroxide groups in an environment having water molecules in the surrounding area in the case of a low-temperature treatment such as optical oxidation. The concentration of the hydroxide groups is influenced by the density of oxide molecules finally existing on the surface of the oxide. In that case, the hydroxide groups do not increase beyond that limit even by irradiation of the light beyond a certain upper time limit. If the temperature is raised, the hydroxide groups once formed decrease through the dewatering condensation through being heated. Although the amount of the hydroxide groups is increased by immersing the inorganic oxide body into water, such requires a long time. In view of the above, it is understood that high temperatures are not preferred.

For example, when apatite hydroxide is to be formed as an inorganic material on the surface of the inorganic oxide body, a simulated body liquid, for example, a mixed aqueous solution containing NaCl, KCI, CaCl₂, MgCl₂ and NaH₃PO₄ and (CH₂OH)₃CNH₂ is used as the reactive solution. At that time, merely immersion of the organic oxide body into the mixed aqueous solution can precipitate apatite hydroxide on the surface of the inorganic oxide body due to the presence of a large amount of the hydroxide on the surface of the inorganic oxide body.

### (Examples)

The present invention will be concretely explained in more detail with reference to Examples, but the invention is not intended to be interpreted as being limited to Examples.

### Example 1

A liquid raw material mixture was prepared by mixing a three-block copolymer : polyethylene oxide-polypropylene oxide-polyethylene oxide (molecular weight of 5800, P-123 manufactured by BASF), tetraethoxy silane (TEOS), hydrochloric acid (HCl) and water (H₂O) at a mixing molecular ratio of 0.017 : 1 : 6 ; 167. A film of a polymer-silica composite material was formed on a substrate made of a titanium alloy by a spin casting method in a thickness of 0.1 or 1 µm. The resulting sample was placed in a vacuum container, and subjected to irradiation with vacuum ultraviolet rays at 172 nm under pressure of 10 Pa for 3 hours (Photocalcination). The intensity of the eximer lamp used was 10 mW/cm². The block copolymer inside the composite body was photochemically removed, thereby forming a silica films having mesopores bored therein.

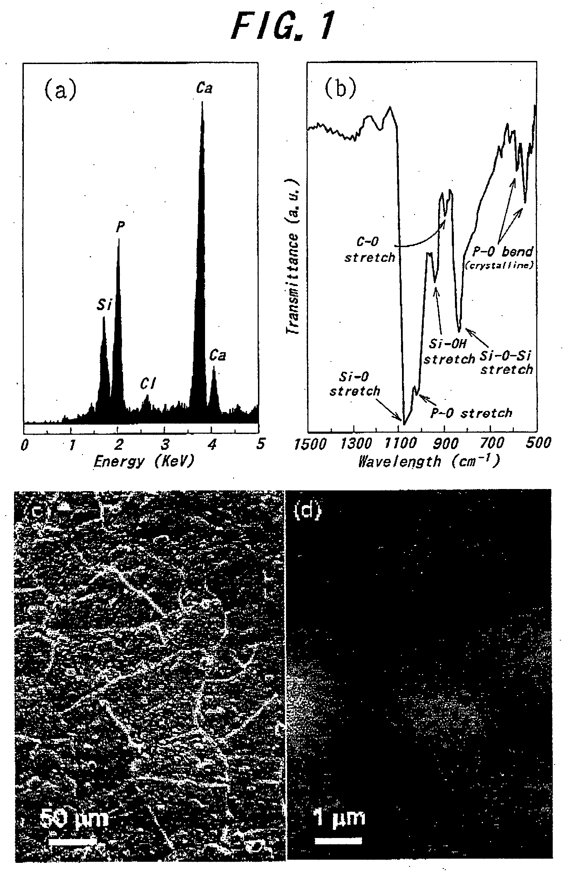

The photochemically oxidized sample was immersed into a simulated body liquid (a mixed aqueous solution containing NaCl, KCl, CaCl₂, MgCl₂, NaH₃PO₄ and (CH₂OH)₃CNH₂ and having a pH value of 7.25 adjusted with HCl) at 35°C for 7 days. The sample taken out was washed, dried, and observed. Results are shown in Fig. 1. In this case, the concentrations of NaCl, KCl, CaCl₂, MgCl₂, NaH₃PO₄ and (CH₂OH)₃CNH₂ were the same as given in the foregoing paragraph 0017.

Figs. 1(a) and 1(b) are an EDX spectrum and an FTIR spectrum of the sample, showing that apatite hydroxide is precipitated upon a surface of the sample. Figs. 1(c) and 1(d) are electron micrograms of the sample, showing that the surface of the sample is covered with apatite crystals without any space.

In Figs. 1(c) and 1(d), white lines and white portions are cracked portions.

As compared with ordinary silica, the mesoporous silica produced by optical oxidation exhibits excellent living activity, and a film of apatite hydroxide was formed on the surface of the sample through being immersed in the simulated body liquid for 7 days only. Adhesion between the silica and the titanium alloy as the substrate is sufficient.

### Example 2

In the same manner as in Example 1, a polymer-silica composite film was formed on a substrate of an titanium alloy. That is, the precursor for an active material was prepared.

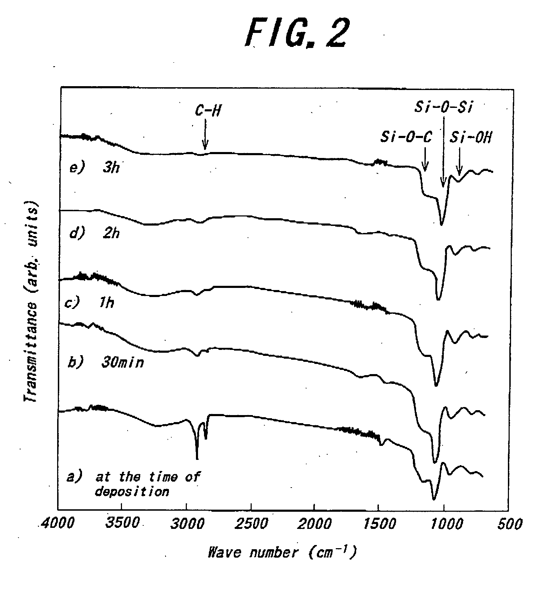

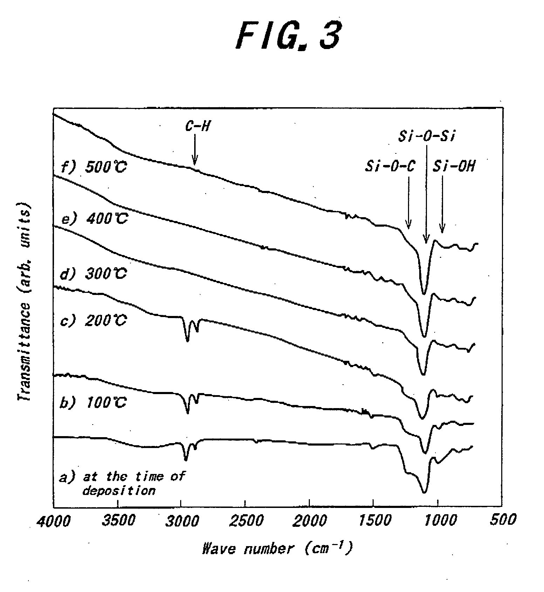

The thus obtained activated material precursor was compared with a conventionally thermally treated precursor for an active material. Results are shown in Figs. 2 and 3. Fig. 2 gives an IR spectrum of the precursor for the active material according to the present invention. Fig. 3 gives an IR spectrum of a precursor of an active material in Comparative Example. In Comparative Example, the precursor was obtained by heating a film of a polymer-silica composite material, as prepared in Example 1, in a temperature range of 400 to 600°C in an oxygen-containing atmosphere, ordinarily in air to burn out the organic material. On the other hand, the organic material is removed by optical oxidation.

As a result, it is seen that almost no hydroxide groups of the precursor for the active material were not observed in the case of the heat treatment.
To the contrary, the precursor for the active material according to the present invention has a clear peak of the hydroxyl groups.

When the precursor for the active material was immersed into the simulated body liquid (the mixed aqueous solution containing NaCl, KCl, CaCl₂, MgCl₂, NaH₃PO₄ and (CH₂OH)₃CNH₂ and having a pH value of 7.25 adjusted with HCI) at 35°C for 7 days, favorable apatite hydroxide was precipitated on the active material obtained.

Since the precursor for the active material according to the present invention has a large amount of the hydroxide groups, it exhibits an advantageous effect that the active material having higher activity can be produced from the precursor.

The activated material according to the present invention has an advantageous effect that it has high activity, and is less susceptible to cracking or breakage.

The active material-producing method according to the present invention has an advantageous effect that apatite hydroxide is precipitated upon the precursor by merely immersing it in the simulated body liquid.

The active material-producing method according to the present invention further has another advantageous effect that it gives good adhesion between the precursor and the substrate.

The active material-producing method according to the present invention has a further advantageous effect that the entire producing steps can be effected near room temperature and therefore the invention method may be applied to plastic materials having no heat resistance.

The active material-producing method according to the present invention has a still further advantageous effect that if the active material is buried in a living body as a substitute part for a fractured bone, a new bone can grow around the active material so that the material may spontaneously fuse to the original bone.

## Claims

1. A precursor for an active material, said precursor comprising an inorganic oxide and having hydroxide groups on a surface thereof
said hydroxide group being in such an amount as to precipitate an inorganic component in a reactive solution containing said inorganic component when the precursor is immersed in the solution.

2. The precursor set forth in claim 1, which is a porous body.

3. The precursor set forth in claim 1 or 2, wherein said inorganic oxide is a ceramic material or a precursor for said ceramic material.

4. The precursor set forth in claim 3, wherein the ceramic material or the ceramic material precursor is at least one selected from the group consisting of a silica-based material, a titania-based material and an alumina-based material.

5. The precursor set forth in any one of claims 1 to 4, wherein the precursor is formed in a filmy shape.

6. The precursor set forth in any one of claims 1 to 5, wherein the inorganic oxide is obtained by removing an organic material from an inorganic oxide-an organic molecular structure by optical oxidation.

7. The precursor set forth in any one of claims 1 to 6, wherein said reactive solution is a aqueous mixed solution of NaCl, KCl, CaCl₂, MgCl₂, NaH₃PO₄ and (CH₂OH)₃CNH₂.

8. The precursor set forth in any one of claims 1 to 7, wherein said inorganic component is apatite hydroxide.

9. An active material comprising the precursor of the active substance in any one of claims 1 to 8 and an inorganic material formed on a surface of said precursor by immersing the precursor into the reactive solution.

10. A method for producing an active material, comprising the steps of forming an inorganic oxide-organic molecular structure by mixing an inorganic oxide with an organic material, selectively removing the organic material of said structure by optical oxidation, immersing a precursor of said active material comprising the inorganic oxide obtained thereby into a reactive solution, and thereby forming an inorganic material on a surface of said inorganic oxide.

11. The method set forth in claim 10, wherein said optical oxidation is effected by irradiation with at least one of ultraviolet rays under vacuum, ozone and plasma.

12. The method set forth in claim 11, wherein said optical oxidation is effected with said ultraviolet rays under vacuum.

13. The method set forth in any one of claims 10 to 12, wherein said reactive solution is a aqueous mixed solution of NaCl, KCl, CaCl₂, MgCl₂, NaH₃PO₄ and (CH₂OH)₃CNH₂.

14. The method set forth in any one of claims 10 to 13, wherein said inorganic material is apatite hydroxide.

15. The method set forth in any one of claims 10 to 14, wherein said inorganic material is a ceramic material or a precursor of the ceramic material.

16. The method set forth in any one of claims 10 to 15, wherein said ceramic material or the ceramic material precursor is at least one selected from the group consisting of a silica-based material, a titania-based material and an alumina-based material.

17. The method set forth in any one of claims 10 to 16, wherein said precursor is formed in a filmy shape.

18. The method set forth in claim 17, wherein the filmy precursor is formed by at least one method selected from the group consisting of a sol-gel method, a CVD method and a chemical deposition method.
